# EUROPEAN PATENT SPECIFICATION

(11) **EP 2 142 933 B1**
(45) Date of publication and mention of the grant of the patent: **02.11.2011**
(21) Application number: 08750051.8
(22) Date of filing: 05.05.2008
(51) Int. Cl.: G01N 33/68

(54) **MULTI-IMMUNOAFFINITY BASED ANTIGEN IDENTIFICATION**
AUF MULTI-IMMUNOAFFINITÄT BASIERENDE INDENTIFIZIERUNG VON ANTIGENEN
IDENTIFICATION D'ANTIGENES BASEE SUR L'IMMUNOAFFINITE MULTIPLE

(30) Priority: 04.05.2007 US 915941 P
(43) Date of publication of application: 13.01.2010
(73) Proprietor: Biosystems International SAS, 91000 Evry (FR)
(72) Inventor: TAKACS, Laszlo, Newbury Park, CA 91320 (US); KADAS, Janos, H-4024 Debrecen (HU); GUTTMAN, Andras, San Diego, CA 92130 (US)
(74) Representative: Becker, Philippe
(86) International application number: PCT/EP2008/055492
(87) International publication number: WO 2008/135553

(56) References cited:
- WO-A-2004/001377
- WO-A-2005/077106
- WO-A-2007/012982
- US-A1- 2003 036 095
- AZARKAN ET AL: "Affinity chromatography: A useful tool in proteomics studies" JOURNAL OF CHROMATOGRAPHY B: BIOMEDICAL SCIENCES & APPLICATIONS, ELSEVIER, AMSTERDAM, NL, vol. 849, no. 1-2, 7 April 2007 (2007-04-07), pages 81-90, XP022024428 ISSN: 1570-0232

## Description

The present invention relates to methods for identifying antigens from (complex) analyte samples. More specifically, the invention relates to methods for identifying antigens recognized by monoclonal antibodies in a (complex) analyte sample.

### GENERAL INTRODUCTION

Generation of bioactive solid surfaces through immobilization of antibodies is important for biomarker discovery and screening. The solid-phase environment provides sufficient bioactivity, stability and reproducibility without a high background (nonspecific binding) or loss of antigens. The progress in microfabrication technologies and the trend towards the creation of integrated biodevices imposes a new and major constrain on immobilization techniques, for example, the requirement for highly defined space-programming of the immobilization of biomolecules. These will be used in high-throughput screening (HTS) processes such as for protein ID of cognate antigens of mAbs from libraries specific for individual elements of complex analytes like the normal human plasma or plasma from diseased subject.

The problem of high throughput cognate antigen protein IDs of mAbs from libraries of mAbs, such as libraries specific for the normal or disease human plasma is that the majority of proteins represent no interest, yet these proteins contaminate the purified analyte of interest and therefore reduce the success rate of the MS based or other identification processes.

Here, we present a novel method that allows the identification of desired antigens from complex analyte samples. The invention more specifically provides novels methods for treating complex samples, that permit antigen isolation and/or identification. The methods of this invention use a two step procedure; first affinity chromatography via a "multiaffinity" step that contains minimum one or a set of mAbs as capture reagents, then, second, "singleaffinity" step that implements parallel affinity chromatography. The product of the first step is loaded onto the second step. Comparative analysis of composition of the flow through and eluates from the second step process allows identification of analyte species that represent specifically depleted and corresponding (e.g. shared physicochemical characteristics such as molecular mass) specifically enriched components that are unique to a given mAb, present as a capture reagent on the second affinity chromatography step. Physicochemical characteristics based separation method such as SDS gel-electrophoresis is used then to further purify the mAb specific components which are then submitted for mass spectrometry based protein identification.

Both the "fullscale" and the "miniaturized" nanoscale and microfluidics based versions of the process are subject of the present patent application.

### SUMMARY OF THE INVENTION

The present invention relates to methods for identifying and/or isolating proteins as defined on the claims. The invention particularly relates to methods of identifying antigens that are bound by antibodies of interest. The invention resides, in particular, in a process which comprises subjecting a sample to be analysed to a first multiaffinity binding step and, then, to at least one singlyaffinity binding step. The method is particularly advantageous in identifying, from complex samples, antigens recognized by antibodies of interest.

### BRIEF LEGEND TO THE FIGURES

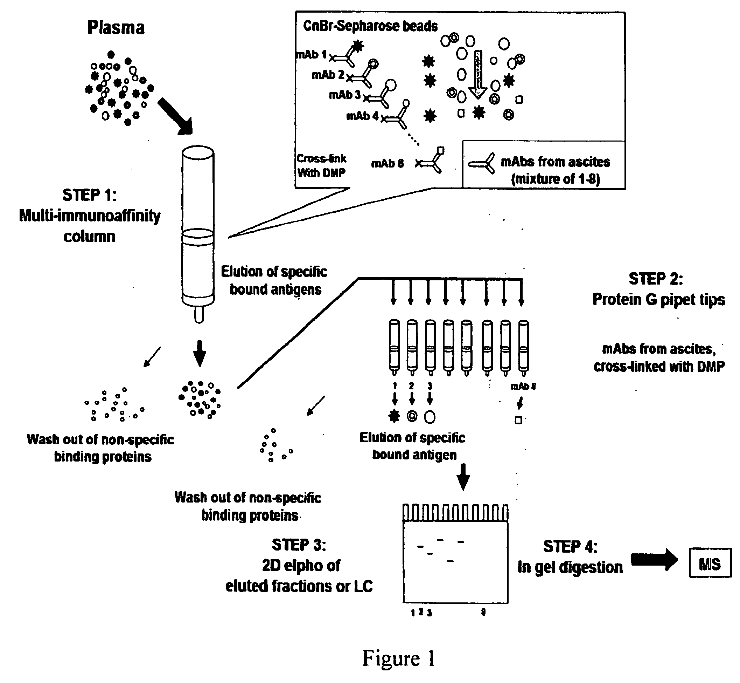

Figure 1: Steps of the sequential identification of each individual fragment from a digested affinity purified analyte species. The identification of the mass of the fragments permits high fidelity protein ID assignment with the use of current empiric and predicted protein data sets.

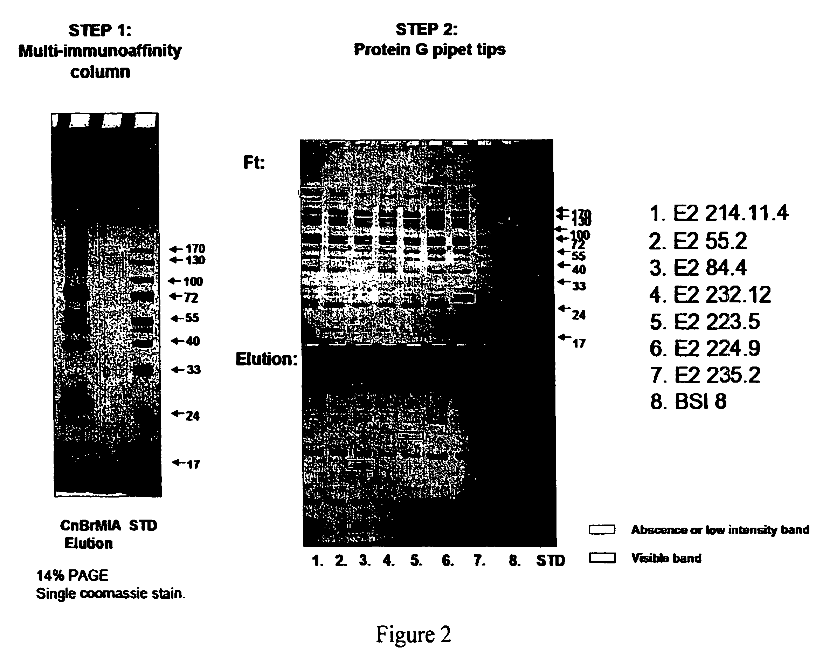

Figure 2. Composition analysis via SDS gel electrophoresis of multiaffinity coloum eluate (Step 1) and single affinity column array flow through and eluates (Step 2). Bands shown in boxes are specific for individual mAbs (listed 1-8).

### DETAILED DESCRIPTION OF THE INVENTION

The invention relates to a method of protein isolation and/or identification comprising the steps of:
a) contacting a sample containing proteins (preferably at least part of said proteins being unknown or uncharacterized) with a multi-affinity column comprising a plurality (e.g., from 3 to 50, from 5 to 40, from 5 to 30, from 5 to 20) of binding reagents;
b) recovering the material bound to said multi-affinity column;
c) separately contacting said material with a plurality of single-affinity columns, each of said single-affinity columns comprising a single binding reagent as contained in the multi-affinity column of step a); and
d) isolating and/or identifying a protein that binds to a binding reagent in at least one of said single-affinity columns.

Preferably, the sample is a complex sample comprising a plurality of proteins, such as a biological fluid, e.g., plasma, blood, serum, bronchoalveolar fluid, urine, sputum, exudates, particularly of human origin. The sample may also be selected from human biopsy material, human tissue section, human faeces, etc., more generally from any material comprising a mixture of proteins. The sample may be pre-treated, e.g., to normalize or reduce the complexity of the sample components.

Preferably, the binding reagents are specific for components of a complex biological fluid, such as human plasma. The binding reagents are preferably monoclonal antibodies, or derivatives thereof (e.g., Fab fragments, ScFv, etc.) having essentially the same antigen specificity.

The supports are chromatography supports, being columns.

In a specific embodiment, the sample is human plasma or serum, and the binding reagents are monoclonal antibodies.

The invention is particularly suited for isolating and/or identifying antigens that are bound by monoclonal antibodies.

The first multiaffinity step is preferably performed using a multiaffinity column prepared as follows. One or, most preferably, a plurality of distinct antibodies (e.g., from 5 to 20) are immobilized on a suitable affinity column. The antibodies are typically immobilized through their Fc portion, more preferably by covalent linkage or crosslinking. The antibodies may be monoclonals, e.g., purified or in the form of hybridoma supernatant, ascites, serum or fermentation fluid. In a preferred embodiment, a plurality of, typically 5-20 different purified mAbs or mAbs from hybridoma supernatant are loaded and crosslinked via their Fc portion onto an appropriate affinity chromatography column (e.g., HiTrap Protein G, Protein -L or CNBr activated Sepharose) allowing the rest of the hybridoma supernatants, or non bound mAbs to exit the system (Multi-Immuno-Affinity (MIA) column preparation).

To perform the multiaffinity step, the appropriate amount of sample (antigen-containing media, such as human plasma) is loaded on the column. In a preferred embodiment, the sample is either pre-treated and/or flown through a mock irrelevant Ab containing column, to remove high abundant proteins prior to be loaded onto the MIA column. This step supports affinity binding of the corresponding antigens and decreased nonspecific interactions.

The affinity column is then washed extensively to remove any remaining non-specifically bound species, and the bound antigens are eluted from the mAb(s) covered affinity surface.

The second step of specific purification of individual antigens by single affinity (microcolumn) array technology is typically performed as follows :
Each individual mAb is load and crosslinked via their Fc portion onto an affinity microcolumn array (e.g., Protein G, Protein L pipet tips), allowing the rest of the hybridoma supernatants to exit the system. An appropriate portion of the product obtained from step 1 is then loaded onto the individual columns/tips of the microcolumn array. This step supports affinity binding of corresponding antigen only in a low microliter scale. In a particular embodiment, the product os step 1) is subjected to an ELISA or dot-blot assay as disclosed in WO/2006/043179, prior to step2. The bound antigens are then eluted from the individual affinity microcolumn array columns.

In a preferred embodiment, the protein identification step comprises subjecting an eluate of at least one of said single-affinity chromatography columns to an electrophoresis, and isolating a protein band of interest. Most preferably, both the eluate and flow through of said at least one single-affinity chromatography column are subjected to electrophoresis, and a protein band that is specific or amplified in the eluate as compared to the flow through is isolated.

Preferred specific steps for said identification step are disclosed below:
1. Slab gel electrophoresis of the individual flow through and eluate samples from the affinity microcolumn array;
2. Comparative analysis for the identification of mAb specific depleted (flow through) and corresponding enriched (eluate) composition (e.g. stained gel images);
3. After visualization, cut off bands of interest;
4. In gel digestion of cutoff bands, sample preparation, digestion and nanoLC-MS analysis to provide protein ID.

The invention may be used, e.g., for identifying antigens characteristic of a disease or trait in a mammal, preferably a human. In this regard, a particular object of this invention resides in a method for isolating and/or identifying antigens specific for a disease or trait of a mammal, comprising the steps of (i) producing antibodies specific for components of a fluid from said mammal and (ii) identifying antigens recognized by said antibodies using a method as described above.

As disclosed in the experimental section, the invention allows the rapid and reliable determination and characterization of antigens recognized by antibodies generated against human plasma. In this regard, the invention has been used to determine the antigen bound by several monoclonal antibodies of interest and the results are listed in Tables 1 and 2, showing the effectiveness of the claimed method.

The disclosure further relates to the use of monoclonal antibody E2 214.11.4, or a derivative thereof (e.g., Fab fragment, CDR region, ScFv, etc.) for detecting the presence or amount of Complement C4-A precursor in a sample, particularly a biological fluid.

The disclosure also encompasses the use of monoclonal antibody E2 84.4, or a derivative thereof (e.g., Fab fragment, CDR region, ScFv, etc.) for detecting the presence or amount of Haptoglobin-related protein/ Haptoglobin in a sample, particularly a biological fluid.

The disclosure further relates to the use of monoclonal antibody E2 223.5, or a derivative thereof (e.g., Fab fragment, CDR region, ScFv, etc.) for detecting the presence or amount of C4b-binding protein alpha chain precursor in a sample, particularly a biological fluid.

The disclosure also resides in the use of monoclonal antibody E2 235.2, or a derivative thereof (e.g., Fab fragment, CDR region, ScFv, etc.) for detecting the presence or amount of Complement C1q subcomponent subunit B precursor in a sample, particularly a biological fluid.

The disclosure also relates to a binding reagent selected from monoclonal antibodies E2 214.11.4, E2 84.4, E2 223.5 and E2 235.2, or derivatives thereof (e.g., Fab fragment, CDR region, ScFv, etc.) having essentially the same antigen specificity.

Further aspects and advantages of this invention will be disclosed in the following examples, which shall be considered as illustrative only and do not limit the scope of the present application.

### EXAMPLES

### Example-1. Multi Immunoaffinity Column chromatography

### Bead: CNBr-activated Sepharose 4B (Coupling capacity: 25-60 mg α-chymotrypsinogen/ml drained medium pH stability: 2-11)

1. Media preparation: 1 g lyophilized powder gives about 3.5 ml final volume of medium, and 5-10 mg protein ligand per ml medium is recommended.
- Weigh out the required amount of powder, and suspend it in 1 mM HCl 2 g powder, gives 7 ml final volume of medium
- Wash the medium washed for 15 minutes with 1 mM HCl on a sintered glass filter Approximately 200 ml 1 mM HCl per gram freeze-dried powder needed, in several aliquots.

### 2. Coupling and blocking

- Dissolve the ligand to be coupled in coupling buffer
   Buffer: 0.1 M NaHCO3 pH 8.3, 0.5 M NaCl
   About 5 ml coupling solution/g lyophilized powder is recommended.
   5-10 mg protein ligand per ml medium is recommended.
   Ligand: 2-2 mg purified IgG from ascites (aliquoted in PBS, 10% glycerol)
   (E2 214.11.4; E2 55.2; E2 84.4; E2 232.12; E2 223.5; E2 224.9; E2 235.2; BSI 8)
- Add the coupling solution containing the ligand with the prepared medium suspension in a stoppered vessel.
- Rotate the mixture end-over-end overnight at 4 °C.
   Other gentle stirring methods may be employed.
- Wash away excess ligand with at least 5 medium (gel) volumes of coupling buffer 35 ml all Buffer: 0.1 M NaHCO3 pH 8.3, 0.5 M NaCl
- Block any remaining active groups. Transfer the medium to a quenching buffer. Let it stand for 15 minutes at 4 °C. Buffer: 1 M ethanolamine, pH 8.0.
- Wash the medium with at least three cycles of alternating pH. Wash with at least 5 medium volumes of each buffer.
   Buffer I.: 0.1 M sodium acetate, pH 4.0 containing 0.5 M NaCl
   Buffer II.: 0.1 M Tris-HCl, pH 8.0 containing 0.5 M NaCl

### 3. Antigen binding

This step should preferably be performed in the cold room.
- Sample pH should be the same as that of the binding buffer.
   Mix the plasma sample with 2X phosphate binding buffer.
   Binding Buffer: PBS 0.1% Triton X-100, 0,05% Sodium Azide
   Check the final volume after mixing!
   Plasma volume before mixing: 150 ml.
   Working mix: 300 ml
- Filter the sample through a 0.22 µm or 0.45 µm filter. (or also centrifuge the plasma at maximal speed in 50 ml tubes in the Beckman centrifuge for 30 minutes (6000 rpm)) It can help us to prolong the working life of the medium.
- Wash the IgG ligand-medium suspension 2 medium volumes of Binding buffer before mix
- Add the plasma sample to the prepared IgG ligand-medium suspension in a stoppered vessel. At once the mix the whole and rotate!!
- Incubate and rotate the mixture for I day at 4 °C.
- Before packing remove the plasma sample from medium, and wash several times (5x) with Binding buffer via resuspending the column in 50ml binding buffer and centrifugation at 400 rpm for 5 minutes in the Beckman centrifuge.

### 4. Packing

Empty column: *GE Amersham XK 16*/*20* (with 1 adaptor, and thermostat jacket) Bed volume: changeable, 5-31 ml; Bed height: changeable, 2,5-15 cm
- Prepare slurry with binding buffer; see below, in a ratio of 75% settled medium to 25% buffer. The binding buffer should not contain agents which significantly increase the viscosity.
- Package the media after washing steps (see washing step at Binding)
- Equilibrate all material to the temperature at which the chromatography will be performed.
- De-gas the medium slurry.
- Eliminate air from the column dead spaces by flushing the end pieces with buffer. Make sure no air has been trapped under the column net. Close the column outlet with a few centimetres of buffer remaining in the column.
- Pour the slurry into the column in one continuous motion. Pouring the slurry down a glass rod held against the wall of the column will minimize the introduction of air bubbles.
- Immediately fill the remainder of the column with buffer, mount the column top piece onto the column and connect the column to a pump.
- Open the bottom outlet of the column and set the pump to run at the desired flow rate. This should be at least 133% of the flow rate to be used during subsequent chromatographic procedures. If I have packed at the maximum linear flow rate, do not exceed 75% of this in subsequent chromatographic procedures. For the washing step use 0,3 ml/min, and after for the elution increase to 1 ml/min. In this case 1,5 ml/min is used for package.
- Maintain the packing flow rate for 3 bed volumes after a constant bed height is reached. Using the adaptor:
- After the medium has been packed as described above, close the column outlet and remove the top piece from the column. Carefully fill the rest of the column with buffer to form an upward meniscus at the top.
- Insert the adaptor at an angle into the column, ensuring that no air is trapped under the net. Make all tubing connections at this stage. There must be a bubble-free liquid connection between the column and the pump.
- Slide the plunger slowly down the column so that the air above the net and in the capillary tubings is displaced by the eluent. Valves on the inlet side of the column should be turned in all directions during this procedure to ensure that air is removed.
- Lock the adaptor in position on the medium surface, open the column outlet and start the flow. Pass buffer at packing flow rate until the medium bed is stable. Re-position the adaptor on the medium surface as necessary.

The column is now packed with bound antigens (hopefully) and ready for use.

### 5. Washing

Use a type of detergent to decrease the unspecific binding, like Triton-X100. Make a pH gradient with several step with low salt concentration. Should be performed with cooled buffers and column (7ml column volume).
- Wash the column at least 5 column volumes of Binding buffer, at 0,5 ml/min (1 hour).
- Wash the column with 10-12 volumes of Wash Buffer I, at 0,3 ml/min (4-4,5 hour). Buffer: 140 mM NaCl, 50 mM NaH2PO4, pH 6.0, 0.1% TX-100
- Wash the column with 3-5 volumes of Wash Buffer II, at 0,3 ml/min (1-1,5 hour). Buffer: 140 mM NaCl, 50 mM NaH2PO4, pH 6.0

### 6. Elution:

- Elute the bound fraction with 2 volumes of Elution buffer, at 1 ml/min. Buffer: 0,1 M glycine-HCl, pH 2.0. Fractionation: 1 ml fractions
- Neutralize eluted fractions with Neutralization Buffer; Buffer: 1 M Tris-HCl, pH 9.0

### 7. QC of eluted fractions

Electrophoresis, in gel digestion, digestion in liquid phase, MS from mixed and concentrated fractions from elution. Optional: Electrophoresis and digestion in liquid phase with MS from separated factions.

### 8. Regeneration and storage of column

- Wash the medium with at least three cycles of alternating pH. Wash with at least 3 medium volumes of each buffer.
   Buffer I.: 0.1 M sodium acetate, pH 4.0 containing 0.5 M NaCl
   Buffer II.: 0.1 M Tris-HCl, pH 8.0 containing 0.5 M NaCl
- Re-equilibrate the column in Binding buffer
- Swollen coupled medium should be stored at 4-8 °C in presence of a bacteriostatic agent.

### Example 2. Phynexus chromatography

Bead/column: *1000*+ *PhyTip columns with Protein G resin:* Maximum solution volume of 1000 µL, Protein G resin volume 160 µL; Coupling capacity: ~1000 µg.

### System: Computer controlled 8-channel pipet

### 1. Tip preparing

PhyTip columns with Protein G are stored in Glycerol when shipped from PhyNexus.
- Wash the tips with 1 ml PBS

| | |
|---|---|
| Program: | 900µl intake/expel |
| | 0,5 ml/min |
| | Delay (hold): after intake 30 sec, after expel 30 sec |
| | Cycle: 2x |

- Repeat this step 2 or 3 times with new PBS solution.

### 2. Coupling and blocking

- Ligand: 0,3 mg purified IgG from ascites (aliquoted in PBS, 10% glycerol).
1. E2 214.11.4
2. E2 55.2
3. E2 84.4
4. E2 232.12
5. E2 223.5
6. E2 224.9
7. E2 235.2
8. BSI 8

- Pipet the ligand in 200 µl (up with PBS to 200 µl) suspension into a 96-well plate separately, according to the numbers (In case of bigger volume, aliquot the ligand suspension to 200-200 µl and intake it separately).
- Intake the antibody solution

| | |
|---|---|
| Program: | 200µl intake/expel |
| | 0,25 ml/min |
| | Delay (hold): after intake 60 sec, after expel 10 sec |
| | Cycle: 4x |

- Repeat it times according to the IgG aliquot number.
- Wash away excess ligand with PBS

| | |
|---|---|
| Program: | 900µl intake/expel |
| | 0,5 ml/min |
| | Delay (hold): after intake 30 sec, after expel 30 sec |
| Cycle: | 4x |

- Cross-link the antibodies to the column surface with DMS/DMP solution

| | |
|---|---|
| Program: | 500µl intake/expel |
| | 0,5 ml/min |
| | Delay (hold): after intake 30 sec, after expel 30 sec |
| | Cycle: 4x |
| Buffer: | 150 mM dimethylpimelimidate (DMP) and 150 mM dimethylsuberimidate (DMS) freshly prepared in ice-cold 0.2 M triethanolamine, pH 8.4. |

- Repeat this step with fresh DMP/DMS solution.
- Wash away the DMS/DMP solution with PBS

| | |
|---|---|
| Program: | 500µl intake/expel |
| | 0,5 ml/min |
| | Delay (hold): after intake 30 sec, after expel 30 sec |
| | Cycle: 4x |

- Block any remaining active groups. Transfer the medium to a quenching buffer.

| | |
|---|---|
| Program: | 500µl intake/expel |
| | 0,5 ml/min |
| | Delay (hold): after intake 30 sec, after expel 30 sec |
| | Cycle: 4x |

Buffer:1 M ethanolamine, pH 8.0
- Repeat this step with fresh ethanolamine solution.
- Wash the medium with PBS

| | |
|---|---|
| Program: | 500µl intake/expel |
| | 0,5 ml/min |
| | Delay (hold): after intake 30 sec, after expel 30 sec |
| | Cycle: 4x |

- Repeat this step with new PBS.
- Optional step: "Empty elution". Make the elution step (Step 6) to elute the unbound IgG. After wash with PBS.

### 3. Antigen binding

Should be performed with chilled solutions. CnBr MIA results about ~600 µl concentrated Eluate at 1,5 µg/µl.
- MIA eluate is in 0,1 M glycine-HCl, pH 2.7 neutralized with 1 M Tris-HCl, pH 9.0
   Optional: after buffer change is in PBS.
   Final volume: 100 µl
   Total protein to the phynexus tips: 50 ug/tip

MIA eluate: mixed and concentrated peak fractions from Multi Immunoaffinity Column chromatography.
- Add the MIA eluate separately to the Phynexus tips

| | |
|---|---|
| Program: | 150µl intake/expel |
| | 0,25 ml/min |
| | Delay (hold): after intake 60 sec, after expel 10 sec |
| | Cycle: 5x |

### 5. Washing

Use a type of detergent to decrease the unspecific binding, like Triton-X100 with cooled buffers.
- Wash the column with PBS

| | |
|---|---|
| Program: | 900 µl intake/expel |
| | 0,5 ml/min |
| | Delay (hold): after intake 30 sec, after expel 10 sec |
| | Cycle: 4x |

- Wash the column with Wash Buffer I
Buffer: 140 mM NaCl, 50 mM NaH2PO4, pH 6.0, 1% TX-100

| | |
|---|---|
| Program: | 900 µl intake/expel |
| | 0,5 ml/min |
| | Delay (hold): after intake 30 sec, after expel 10 sec |
| | Cycle: 4x |

- Repeat this step 4 times with new buffer.
- Wash the column with Wash Buffer II
Buffer: 140 mM NaCl, 50 mM NaH2PO4, pH 6.0

This step is for to remove the residual Tx-100 from the column.

| | |
|---|---|
| Program: | 900 µl intake/expel |
| | 0,5 ml/min |
| | Delay (hold): after intake 30 sec, after expel 10 sec |
| Cycle: | 4x |

### 6. Elution:

- Elute the bound fraction with 100 µl Elution buffer.

| | |
|---|---|
| Buffer: | 0,1 M glycine-HCl, pH 2.0 |
| Program: | 100 µl intake/expel |
| | 0,5 ml/min |
| | Delay (hold): after intake 20 sec, after expel 10 sec |
| | Cycle: 4x |

- Neutralize eluted fractions with 20 µl Neutralization Buffer
Buffer: 1 M Tris-HCl, pH 9.0

7. Composition analysis of eluted and flow through fractions: Electrophoresis, comparison (e.g. automated image analysis) in gel digestion, digestion in liquid phase, MS.

### 8. Regeneration and storage of column

- Wash the column with PBS

| | |
|---|---|
| Program: | 900µl intake/expel |
| | 0,5 ml/min |
| | Delay (hold): after intake 30 sec, after expel 30 sec |
| | Cycle: 4x |

- Wash and store the column in PBS,

| | |
|---|---|
| Program: | 900µl intake/expel |
| | 0,5 ml/min |
| | Delay (hold): after intake 30 sec, after expel 30 sec |
| | Cycle: 4x |

- Phynexus tips should be stored at 4-8 °C in presence of a glycerol

**Table 1:**

| Output of MS based protein ID (GenBank accession number, gene names, predicted Mw., confidence level and number of peptides) of material out from boxed areas of the SDS PAGE gels of eluted samples. | | | | | |
|---|---|---|---|---|---|
| 20070425IgG1 | | | | | |
| **Accession #** | **Name** | | **Confidence** | **Peptipe Found** | **Biological Process** |
| gi\|81175238 | Complement C4-A precursor (Acidic complement C4) | **192771 Da** | | 99 | 3 Serum |
| gi\|81175167 | Complement C4-B precursor (Basic complement C4) | **192793 Da** | | 99 | 3 Serum |
| | | | | | |
| **20070425IgG3A** | | | | | |
| Accession # | Name | | Confidence | Peptide Found | Biological Process |
| gi\|123508 | Haptoglobin precursor | **45205 Da** | | 99 | 11 Serum |
| gi\|123510 | Haptoglobin-relaled protein precursor | **39008 Da** | | 99 | 8 Secreted (potential) |
| | | | | | |
| **20070425IgG3B** | | | | | |

| Accession # | Name | | Confidence | Peptide Found | Biological Process |
|---|---|---|---|---|---|
| gi\|123508 | Haptoglobin precursor | **45205 Da** | | 99 | 7 Serum |
| gi\|123510 | Haptoglobin-related protein precursor | **39008 Da** | | 99 | 4 Serum |
| | | | | | |
| **20070425IgG5** | | | | | |

| Accession # | Name | | Confidence | Peptide Found | Biological Process |
|---|---|---|---|---|---|
| gi\|416733 | C4b-binding protein alpha chain precursor (C4bp) | **67033 Da** | | 99 | 2 Serum |
| | | | | | |
| **20070425IgG7** | | | | | |

| Accession # | Name | | Confidence | Peptide Found | Biological Process |
|---|---|---|---|---|---|
| gi\|399140 | Complement C1q subcomponent subunit B precursor | **26459 Da** | | 99 | 4 Serum |

**Table 2:**

| Protein IDs of mAbs 1, 3, 5 and 7. | |
|---|---|
| New IDs: | |
| 1. E2 214.11.4 | - Complement C4-A precursor |
| 3. E2 84.4 | - Haptoglobin-related protein/ Haptoglobin |
| 5. E2 223.5 | - C4b-binding protein alpha chain precursor |
| 7. E2 235.2 | - Complement C1q subcomponent subunit B precursor |

## Claims

1. A method of protein isolation and/or identification comprising the steps of:
a) contacting a sample containing proteins with a multi-affinity chromatography column comprising a plurality of binding reagents;
b) recovering the material bound to said multi-affinity chromatography column;
c) separately contacting said material with a plurality of single-affinity chromatography columns, each of said single-affinity chromatography columns comprising a single binding reagent as contained in the multi-affinity chromatography column of step a); and
d) isolating and/or identifying a protein that binds to a binding reagent in at least one of said single-affinity chromatography columns.

2. The method of claim 1, wherein the sample is a complex sample comprising a plurality of proteins, such as a biological fluid, e.g., plasma, blood, serum, particularly of human origin.

3. The method of claim 2, wherein the sample is pre-treated to normalize or reduce the complexity of the sample components.

4. The method of any one of claims 1 to 3, wherein the binding reagents are specific for components of a complex biological fluid, such as human plasma.

5. The method of any one of claims 1 to 4, wherein the binding reagents are monoclonal antibodies, or derivatives thereof having essentially the same antigen specificity.

6. The method of any one of claims 1 to 5, wherein the sample is human plasma or serum, and the binding reagents are monoclonal antibodies.

7. The method of any one of claims 1 to 5, wherein the multiaffinity chromatography column is prepared by loading and crosslinking a plurality of different purified mAbs or mAbs from hybridoma supernatant via their Fc portion onto an appropriate affinity chromatography column, preferably selected from HiTrap Protein G, Protein -L and CNBr activated Sepharose, and allowing the rest of the hybridoma supernatants, or non bound mAbs, to exit the system.

8. The method of any one of claims 1 to 7, wherein the multiaffinity chromatography step is performed by loading the appropriate amount of sample on the column; extensive washing to remove any remaining non-specifically bound species, and eluting the bound antigens from the mAb(s) covered affinity surface.

9. The method of any one of claims 1 to 8, wherein the single affinity column is prepared by loading and crosslinking each individual mAbs via their Fc portion onto an affinity microcolumn array, preferably selected from Protein G and Protein L pipet tips.

10. The method of claim 9, wherein the single affinity chromatography step is performed by loading an appropriate portion of the product of step 1 onto the individual columns/tips of the microcolumn array and eluting the bound antigens from the individual affinity microcolumn array columns.

11. The method of any one of claims 1 to 10, wherein the protein identification step comprises subjecting an eluate of at least one of said single-affinity chromatography columns to an electrophoresis, and isolating a protein band of interest.

12. The method of claim 11, wherein both the eluate and flow through of said at least one single-affinity chromatography column are subjected to electrophoresis, and a protein band that is specific or amplified in the eluate as compared to the flow through is isolated.

13. The method of any one of claims 1 to 12, wherein said identification step comprises:
1. Slab gel electrophoresis of the individual flow through and eluate samples from the affinity microcolumn array;
2. Comparative analysis for the identification of mAb specific depleted (flow through) and corresponding enriched (eluate) composition;
3. After visualization, cut off bands of interest;
4. In gel digestion of cutoff bands, sample preparation, digestion and nanoLC-MS analysis to provide protein ID.

14. The method of any one of claims 1 to 13, for identifying antigens that are bound by monoclonal antibodies.

15. The method of any one of claims 1 to 14, for identifying antigens characteristic of a disease or trait in a mammal, preferably a human.

16. A method of identifying antigens specific for a disease or trait of a mammal, comprising the steps of (i) producing antibodies specific for components of a fluid from said mammal and (ii) identifying antigens recognized by said antibodies using a method of any one of claims 1 to 13.

## Patentansprüche

1. Ein Verfahren zur Protein-Isolierung und/oder Identifizierung umfassend die Schritte:
a) in Kontakt Bringen einer Probe enthaltend Proteine mit einer Multi-Affinitätschromatographiesäule umfassend eine Vielzahl von Bindungsreagenzien;
b) Gewinnen des an diese Multi-Affinitätschromatographiesäule gebundenen Materials;
c) getrenntes in Kontakt Bringen dieses Materials mit einer Vielzahl von Einzel-Affinitätschromatographiesäulen, wobei jede dieser Einzel-Affinitätschromatographiesäulen ein einzelnes Bindungsreagenz wie in der Multi-Affinitätschromatographiesäule nach Schritt a) umfasst; und
d) Isolieren und/oder Identifizieren eines Proteins, das an ein Bindungsreagenz in mindestens einer dieser Einzel-Affinitätschromatographiesäulen bindet.

2. Das Verfahren nach Anspruch 1, wobei die Probe eine komplexe Probe ist, die eine Vielzahl von Proteinen, wie eine biologische Flüssigkeit, z.B. Plasma, Blut, Serum, insbesondere menschlichen Ursprungs, umfasst.

3. Das Verfahren nach Anspruch 2, wobei die Probe vorbehandelt ist, um die Komplexität der Probenbestandteile zu normalisieren oder reduzieren.

4. Das Verfahren nach einem der Ansprüche 1 bis 3, wobei die Bindungsreagenzien spezifisch für die Bestandteile einer komplexen biologischen Flüssigkeit, wie menschliches Plasma, sind.

5. Das Verfahren nach einem der Ansprüche 1 bis 4, wobei die Bindungsreagenzien monoklonale Antikörper oder Derivate davon sind, die im Wesentlichen die gleiche Antigenspezifität haben.

6. Das Verfahren nach einem der Ansprüche 1 bis 5, wobei die Probe menschliches Plasma oder Serum und die Bindungsreagenzien monoklonale Antikörper sind.

7. Das Verfahren nach einem der Ansprüche 1 bis 5, wobei die Multi-Affinitätschromatographiesäule vorbereitet wird durch Beladen und Vernetzen einer Vielzahl verschiedener gereinigter mAbs oder mAbs aus Hybridomüberstand über deren Fc-Anteil an einer geeigneten Affinitätschromatographiesäule, vorzugsweise ausgewählt aus HiTrap Protein G, Protein -L und CNBr aktivierter Sepharose, und Erlauben dem Rest der Hybridomaüberstände oder der nicht gebundenen mAbs das System zu verlassen.

8. Das Verfahren nach einem der Ansprüche 1 bis 7, wobei der Multi-Affinitätschromatographieschritt durch Beladen der geeigneten Menge der Probe auf die Säule; ausführliches Waschen, um irgendeine verbleibende nicht spezifisch gebundene Art zu entfernen, und Eluieren des gebundenen Antigens von mit mAb(s) bedeckten Affinitätsoberfläche durchgeführt wird.

9. Das Verfahren nach einem der Ansprüche 1 bis 8, wobei die Einzel-Affinitätssäule vorbereitet wird durch Beladen und Vernetzen jedes einzelnen der mAbs über ihren Fc-Anteil an einem Affinitätsmikrosäulenarray, vorzugsweise ausgewählt aus Protein G und Protein L Pipettenspitzen.

10. Das Verfahren nach Anspruch 9, wobei der Einzel-Affinitätschromatographieschritt durch Beladen eines geeigneten Anteils des Produkts des Schritts 1 auf die individuellen Säulen/Spitzen des Mikrosäulenarray und Eluieren der gebundenen Antigene von dem individuellen Affinitätsmikrosäulenarray durchgeführt wird.

11. Das Verfahren nach einem der Ansprüche 1 bis 10, wobei der Proteinidentifizierungsschritt das Unterwerfen eines Eluats von mindestens einer dieser Einzel-Affinitätschromatographiesäulen einer Elektrophorese und Isolieren einer interessierenden Proteinbande umfasst.

12. Das Verfahren nach Anspruch 11, wobei sowohl das Eluat als auch der Durchfluss dieser mindestens einen Einzel-Affinitätschromatographiesäule einer Elektrophorese unterzogen werden und eine Proteinbande, die in dem Eluat verglichen zu dem Durchfluss spezifisch oder amplifiziert ist, isoliert wird.

13. Das Verfahren nach einem der Ansprüche 1 bis 12, wobei dieser Identifizierungsschritt umfasst:
1. Platten (Slab)-gelelektrophorese der individuellen Durchfluss- und Eluat-Proben aus dem Affinitätsmikrosäulenarray;
2. Vergleichende Analyse zur Identifizierung von mAb spezifisch depletierter (Durchfluss) und entsprechend angereicherter (Eluat) Zusammensetzung;
3. Nach Sichtbarmachung, Ausschnitt der interessierenden Banden;
4. In-Gel-Verdau der ausgeschnittenen Banden, Probenvorbereitung, Verdau und nanoLC-MS Analyse, um die Protein ID bereitzustellen.

14. Das Verfahren nach einem der Ansprüche 1 bis 13 zur Identifizierung von Antigenen, die durch monoklonale Antikörper gebunden sind.

15. Das Verfahren nach einem der Ansprüche 1 bis 14 zur Identifizierung von Antigenen charakteristisch für eine Krankheit oder Eigenschaft in einem Säugetier, vorzugsweise einem Menschen.

16. Ein Verfahren zur Identifizierung von Antigenen spezifisch für eine Krankheit oder Eigenschaft eines Säugetiers, umfassend die Schritte (i) Herstellen von Antikörpern spezifisch für Bestandteile einer Flüssigkeit von diesem Säugetier und (ii) Identifizieren von Antigenen, die durch diese Antikörper erkannt werden, unter Verwendung eines Verfahrens nach einem der Ansprüche 1 bis 13.

## Revendications

1. Méthode d'isolement et/ou d'identification de protéine comprenant les étapes suivantes :
a) la mise en contact d'un échantillon contenant des protéines avec une colonne de chromatographie de multi-affinité comprenant une pluralité de réactifs de liaison ;
b) la récupération du matériel lié à ladite colonne de chromatographie de multi-affinité ;
c) la mise en contact séparément dudit matériel avec une pluralité de colonnes de chromatographie d'affinité simple, chacune desdites colonnes de chromatographie d'affinité simple comprenant un réactif de liaison simple comme contenu dans la colonne de chromatographie de multi-affinité de l'étape a) ; et
d) l'isolement et/ou l'identification d'une protéine qui se lie à un réactif de liaison dans au moins une desdites colonnes de chromatographie d'affinité simple.

2. Méthode de la revendication 1, dans laquelle l'échantillon est un échantillon complexe comprenant une pluralité de protéines, tel qu'un fluide biologique, par exemple, du plasma, du sang, du sérum, en particulier d'origine humaine.

3. Méthode de la revendication 2, dans laquelle l'échantillon est prétraité pour normaliser ou réduire la complexité des composants de l'échantillon.

4. Méthode de l'une quelconque des revendications 1 à 3, dans laquelle les réactifs de liaison sont spécifiques pour des composants d'un fluide biologique complexe, tel que le plasma humain.

5. Méthode de l'une quelconque des revendications 1 à 4, dans laquelle les réactifs de liaison sont des anticorps monoclonaux, ou des dérivés de ceux-ci ayant essentiellement la même spécificité antigénique.

6. Méthode de l'une quelconque des revendications 1 à 5, dans laquelle l'échantillon est du plasma ou du sérum humain, et les réactifs de liaison sont des anticorps monoclonaux.

7. Méthode de l'une quelconque des revendications 1 à 5, dans laquelle la colonne de chromatographie de multi-affinité est préparée en chargeant et réticulant une pluralité de mAbs purifiés différents ou mAbs provenant du surnageant d'hybridomes par l'intermédiaire de leur partie Fc sur une colonne de chromatographie d'affinité appropriée, de préférence choisie parmi la Protéine G HiTrap, la Protéine L et la Sépharose activée par CNBr, et en permettant au reste des surnageants d'hybridome, ou des mAbs non liés, de sortir du système.

8. Méthode de l'une quelconque des revendications 1 à 7, dans laquelle l'étape de chromatographie de multi-affinité est exécutée par la charge d'une quantité appropriée d'échantillon sur la colonne ; le lavage extensif pour enlever toute espèce résiduelle liée de façon non-spécifique, et l'élution des antigènes liés de la surface d'affinité couverte par les mAb(s).

9. Méthode de l'une quelconque des revendications 1 à 8, dans laquelle la colonne d'affinité simple est préparée par la charge et la réticulation de chaque mAbs individuels par l'intermédiaire de leur partie Fc sur une rangée de micro-colonnes d'affinité, de préférence choisie parmi des embouts de pipette Protéine G et Protéine L.

10. Méthode de la revendication 9, dans laquelle l'étape de chromatographie d'affinité simple est exécutée en chargeant une partie appropriée du produit de l'étape 1 sur les colonnes/embouts individuels de la rangée de micro-colonnes et en éluant les antigènes liés à partir des colonnes de la rangée de micro-colonnes d'affinité individuelles.

11. Méthode de l'une quelconque des revendications 1 à 10, dans laquelle l'étape d'identification de protéine comprend la soumission d'un éluat d'au moins une desdites colonnes de chromatographie d'affinité simple à une électrophorèse, et l'isolement d'une bande de protéine d'intérêt.

12. Méthode de la revendication 11, dans laquelle l'éluat et le flux d'écoulement de ladite au moins une colonne de chromatographie d'affinité simple sont tous deux soumis à l'électrophorèse, et une bande de protéine qui est spécifique ou amplifiée dans l'éluat par rapport au flux d'écoulement est isolée.

13. Méthode de l'une quelconque des revendications 1 à 12, dans laquelle ladite étape d'identification comprend :
1. Electrophorèse sur une plaque de gel des échantillons de flux d'écoulement et d'éluat individuels à partir de la rangée de micro-colonne d'affinité ;
2. Analyse comparative pour l'identification de composition déplétée en mAb spécifiques (flux d'écoulement) et de composition correspondante enrichie (éluat) ;
3. Après la visualisation, le découpage des bandes d'intérêt ;
4. Digestion des bandes de coupure dans le gel, la préparation des échantillons, la digestion et l'analyse nano LC-MS pour fournir l'identification de protéine.

14. Méthode de l'une quelconque des revendications 1 à 13, pour identifier des antigènes qui sont liés par des anticorps monoclonaux.

15. Méthode de l'une quelconque des revendications 1 à 14, pour identifier des antigènes caractéristiques d'une maladie ou d'un trait chez un mammifère, de préférence chez un humain.

16. Méthode pour identifier des antigènes spécifiques d'une maladie ou d'un trait d'un mammifère, comprenant les étapes suivantes (i) la production d'anticorps spécifiques pour des composants d'un fluide provenant dudit mammifère et (ii) l'identification des antigènes reconnus par lesdits anticorps en utilisant une méthode de l'une quelconque des revendications 1 à 13.
